# EUROPEAN PATENT APPLICATION

(11) **EP 2 023 254 A1**
(43) Date of publication of application: **11.02.2009**
(21) Application number: 06708879.9
(22) Date of filing: 07.02.2006
(51) Int. Cl.: G06F 19/00

(54) **TOOL FOR THE DEAF PROVIDING ACCESS TO A MEDICAL EMERGENCY SERVICE**

(71) Applicant: Asisa Asistencia Sanitaria Interprovincial De Seguros, S.A., 28027 Madrid (ES)
(72) Inventor: CAPILLA SANMARTIN, Aurelio, 28027 Madrid (ES); MORENO LAUREIRO, Domingo, 28027 Madrid (ES); INFANTE RODRIGUEZ, Fernando, 28027 Madrid (ES)
(74) Representative: Esteban Perez-Serrano, Maria Isabel
(86) International application number: PCT/ES2006/070008
(87) International publication number: WO 2007/090909

(57) **Abstract**

The object of the present invention is a tool for accessibility of the deaf to the emergency medical service that eliminates communications barriers and avoids the mediation of third persons in a fully mobile environment by integrating in mobile devices a medical data collection application as well as integrating the mobile device itself in the operation of the emergency service; it also allows determining a preliminary diagnosis by the emergency service in a very reliable manner, due to the simplicity and ease of use of the medical data collection application installed in the patient's mobile device.

## Description

### OBJECT OF THE INVENTION

The present invention relates to a tool for accessibility of the deaf to the medical emergency service that eliminates communications barriers and avoids the mediation of third persons in a fully mobile environment.

The invention characterises the integration mobile devices of a medical data collection application as well as the integration of the mobile device itself in the operation of the emergency service.

This tool also allows determining a preliminary diagnosis by the emergency service in a very reliable manner, due to the simplicity and ease of use of the medical data collection application installed in the patient's mobile device.

### BACKGROUND OF THE INVENTION

Intermediation centres used by deaf persons to request emergency medical care when they are not accompanied by a hearing person are well known.

These elements are reception stations where an operator receives the information arriving from the patient by telephones installed for this purpose at the patient's home.

In these cases, the operator acts as an intermediary between the emergency service and the deaf person, thereby reducing the privacy of the doctor-patient relationship, as well as subjecting the information to the interpretation of the operator.

In addition, due to the large size of these terminals and to the fact that they are permanently installed in the patient's home, it is not possible to transport them from one place to another to make them available to the deaf person at all times.

Lastly, due to the reading and writing problems suffered by many deaf patients, the data collection performed by the operator may lose descriptive detail.

All of the above drawbacks are overcome by the invention described below.

### DESCRIPTION OF THE INVENTION

The present invention relates to a tool for accessibility of the deaf to the emergency medical service that eliminates communications barriers and avoids the mediation of third parties in a fully mobile environment.

This tool allows integrating a graphical, specific and easy-to-use medical data collection tool in mobile devices, allowing the deaf patient to communicate with the emergency service personally, from any place and in a concise manner providing the medical data needed to determine which resources to send as required by the patient.

The medical data collection application presents a graphical environment in which the patient can locate the ailment and the main symptoms, guiding the patient from this point to complete the information required from a medical standpoint.

The deaf patient must have a mobile device, among which are mobile telephones, portable computers and PDAs (personal digital assistant), in which has been downloaded the application that allows the patient to communicate with the reception station located in the emergency service via a mobile applications server.

An emergency specialist physician will be present at said reception station to evaluate the information received. This physician can establish immediate communication with patients via their mobile device.

On another hand, the information obtained from the patient and evaluated by the physician is sent to an SQL (structured query language) database which holds the patient's personal file and medical record, generating a working report that assigns a resource for assisting the patient, as well as sending the patient a message indicating the expected response time and the resource assigned.

Among the assigned resources are solutions by conversation over the Internet with the patient, sending an ambulance for transport to a hospital or sending a physician to the patient's home.

### DESCRIPTION OF THE DRAWING

The present descriptive memory is completed by a set of drawings illustrating the preferred embodiment without limiting the invention.

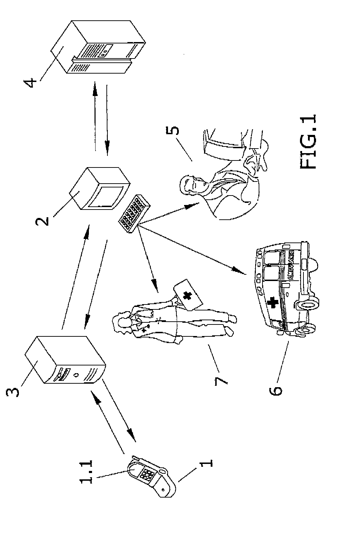

Figure 1 shows a functional scheme of the tool for accessibility of the deaf to the emergency medical service.

### PREFERRED EMBODIMENT OF THE INVENTION

In view of the above, the present invention relates to a tool for accessibility of the deaf to the emergency medical service.

The tool comprises a mobile device (1) in which is downloaded at the time of first use an application (1.1) for collecting medical data, said application requiring an access code known to the patient for operation.

The mobile devices (1) in which the medical data collection application (1.1) is downloaded are preferably a mobile telephone, a portable computer or a PDA.

The medical data collection application (1.1) has a graphical environment generated by a programming language, preferably Java or Symbian, in which the patient locates the ailment and the main symptoms, from which point the patient is guided to enter all the information required from a medical standpoint.

When the patient has entered all the symptoms related to the ailment, disease or accident, the information is transmitted from the mobile device (1) to a reception station (2) located in the emergency service via a mobile applications server (3).

An emergency specialist physician (not shown in figure 1) will be present at said reception station (2) to evaluate the information received. This physician can establish immediate communication with patients via their mobile device (1).

On another hand, the information obtained from the patient and evaluated by the physician is sent to a preferably SQL database (4) which holds the patient's personal file and medical record, generating a working report that assigns a resource (5, 6, 7) for assisting the patient.

Among the assigned resources (5, 6, 7) are solutions by conversation over the Internet (5) with the patient, sending an ambulance for transport to a hospital (6) or sending a physician to the patient's home (7).

Said database (4) also sends a message to the patient indicating the expected response time and the resource (5, 6, 7) assigned.

The essence of this invention is not affected by variations in the materials, shape, size and arrangement of its component elements, described in a nonlimiting manner that should allow its reproduction by an expert.

## Claims

1. Tool for accessibility of the deaf to the emergency medical service of those having a station (2) located in the emergency service for receiving alerts via a mobile applications server (3), wherein the information obtained from the patient and evaluated by a physician is sent to a preferably SQL database (4), which holds a personal file and the medical record of the patient, generating a working report that assigns a resource (5, 6, 7) to assist the patient which can be a solution by establishing a physician-patient conversation over the Internet (5), sending an ambulance (6) for transport to a hospital or sending a physician to the patient's home (7), **characterised in that** the transmission of information by the deaf patient to the emergency medical service is performed by a mobile device (1) that includes a medical data collection application (1.1) with a graphical environment in which the patient locates the ailment and main symptoms and is guided from this point to enter all the information required from a medical standpoint.

2. Tool for accessibility of the deaf to the emergency medical service according to claim 1, **characterised in that** the physician based at the reception station (2) establishes an immediate personal communication with the patient via the mobile device (1).

3. Tool for accessibility of the deaf to the emergency medical service according to claim 1, **characterised in that** the medical data collection application (1.1) requires an access code known to the patient for operation.

4. Tool for accessibility of the deaf to the emergency medical service according to claim 1, **characterised in that** the mobile device (1) is a mobile telephone.

5. Tool for accessibility of the deaf to the emergency medical service according to claim 1, **characterised in that** the mobile device (1) is a portable computer.

6. Tool for accessibility of the deaf to the emergency medical service according to claim 1, **characterised in that** the mobile device (1) is a PDA.

7. Tool for accessibility of the deaf to the emergency medical service according to claim 1, **characterised in that** the database (4) also sends the patient a message indicating the expected response time and the resource (5, 6, 7) assigned.
